# FASCICULE DE BREVET EUROPEEN

(11) **EP 0 757 913 B1**
(45) Date de publication et mention de la délivrance du brevet: **21.11.2001**
(21) Numéro de dépôt: 95203357.9
(22) Date de dépôt: 06.12.1995
(51) Int. Cl.: A61N 1/40

(54) **Générateur d'ondes électromagnétiques**
Generator zur Erzeugung von elektromagnetischen Wellen
Generator for electro-magnetic waves

(30) Priorité: 08.06.1995 ES 9501554 U
(43) Date de publication de la demande: 12.02.1997
(73) Titulaire: Suarez Martinez, Miguel, 31300 Talfalla (Navarra) (ES)
(72) Inventeur: Oses Navaz, Juan, E-31006 Pamplona (Navarra) (ES)
(74) Mandataire: Bruder, Michel

(56) Documents cités:
- EP-A- 0 196 180
- EP-A- 0 522 425
- EP-A- 0 529 620
- US-A- 4 213 185

## Description

La présente invention concerne un générateur d'ondes électromagnétiques utilisable plus particulièrement dans le domaine médical.

Les recherches en médecine biologique ont montré que toutes les fonctions de l'organisme humain s'accompagnent de phénomènes bioélectriques et on peut maintenant affirmer que les cellules internes de l'organisme sont soumises à une influence électromagnétique et que les différents états mentaux correspondent à des fréquences déterminées dans les cellules du cerveau.

De la même façon on a constaté que la fonction électromagnétique de l'intérieur des cellules du corps est influencée directement par les champs biomagnétiques qui les entourent. Ceci a permis de déterminer qu'en se servant de champs magnétiques bien définis, il est possible de modifier l'état électromagnétique corporel afin de produire, en fonction de cet état, la stimulation des états mentaux souhaités.

Par conséquent, un but de la présente invention est de fournir un générateur d'ondes électromagnétiques, à des fréquences déterminées appropriées pour produire des influences capables de produire des états corporels distincts, par exemple pour produire une plus grande relaxation dans un état de repos ou un plus grand dynamisme pour une activité quelconque.

A cet effet, ce générateur d'ondes électromagnétiques est caractérisé en ce qu'il comprend un microprocesseur avec un circuit oscillant formé par un cristal de quartz et deux condensateurs, et, à la sortie de ce microprocesseur, une antenne émettrice formée par une bobine laquelle est reliée à la sortie du microprocesseur par l'intermédiaire d'une résistance limitatrice de courant.

Des générateurs d'ondes electro-magnétiques comportant un micro-processeur sont connus de US-A-4 213 185 mais le microprocesseur n'est pas relié directement à l'antenne par une résistance.

Dans le générateur d'ondes électromagnétiques suivant la présente invention, le circuit oscillant produit la fréquence d'horloge du microprocesseur qui produit à son tour les ondes qui sont sélectionnées au moyen de contacts actionnés à volonté par l'utilisateur. Le microprocesseur émet un signal spécifique, suivant la sélection, afin de donner lieu à une émission d'ondes par l'intermédiaire de la bobine fonctionnant en antenne émettrice.

On obtient ainsi un appareil capable de produire une série de fréquences qui sont sélectionnables d'une façon distincte, afin de créer l'influence magnétique que l'utilisateur désire.

L'appareil a une consommation électrique minimale du fait que l'alimentation pour son fonctionnement exige seulement une pile ou une batterie de 3 volts. En outre, l'appareil se présente sous une forme très réduite, lui permettant d'être utilisé aussi bien en étant placé sur un meuble quelconque, tel que, par exemple, sur une table de nuit située d'un côté d'un lit, qu'en étant logé dans une poche d'un article d'habillement, en vue de son utilisation lorsqu'elle est désirée.

Le champ d'action de l'appareil peut varier en fonction de l'antenne utilisée laquelle présente, suivant une réalisation préférée, une taille réduite correspondant à l'ensemble de l'appareil tout en étant capable de produire un champ d'action dans un volume spatial allant d'un à deux mètres cubes, ce qui est naturellement suffisant pour les formes d'application précitées.

L'appareil suivant la présente invention présente des caractéristiques très avantageuses qui le rendent tout à fait approprié pour la fonction précitée d'adaptation d'un état corporel au moyen d'influences magnétiques.

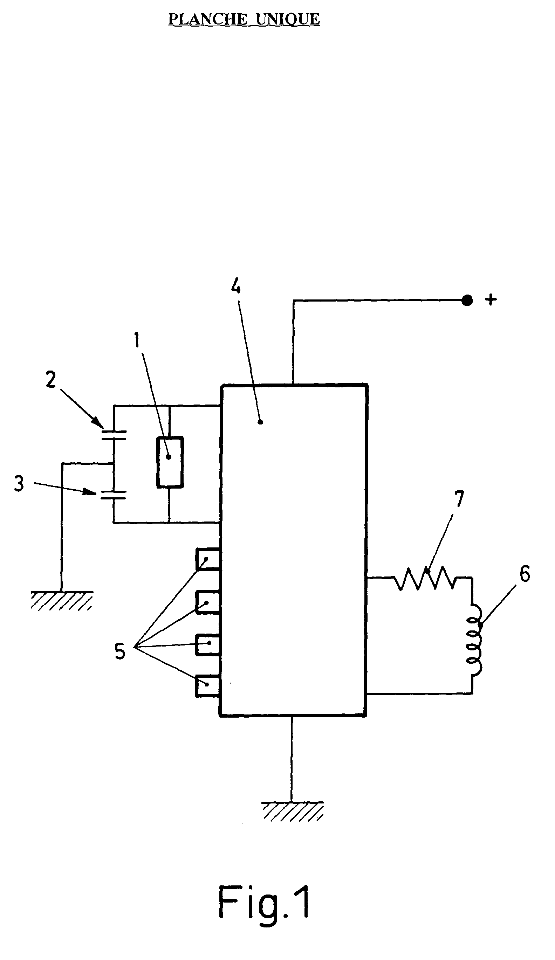

On décrira ci-après, à titre d'exemple non limitatif, une forme d'exécution de la présente invention en référence au dessin annexé sur lequel la figure 1 unique est un schéma d'un appareil générateur d'ondes électromagnétiques.

L'appareil générateur d'ondes électromagnétiques suivant la présente invention, destiné à exercer une influence sur le corps humain, par une adaptation à des états biophysiques concrets sélectionnables par l'utilisateur, comporte un microprocesseur 4 et un circuit oscillant formé par un cristal de quartz 1 de deux condensateurs 2 et 3 branchés en série entre eux et en dérivation sur le cristal 1. Le point de jonction des deux condensateurs 2 et 3 est relié à la masse. Le circuit oscillant 1,2 et 3 produit la fréquence d'horloge pour le fonctionnement correct du microprocesseur 4. Il existe en outre un circuit constitué de diverses résistances, représentées par la résistance limitatrice de courant 7.

Le cristal de quartz 1 oscille à une fréquence de 32 kilohertz puisqu'une vitesse excessive n'est pas nécessaire et ce qui permet cependant une consommation minimale de courant. En ce qui concerne les condensateurs 2 et 3, leur capacité est comprise dans la plage allant de trente-trois picofarads (33 pF) et soixante huit picofarads (68 pF).

Ce circuit oscillant est associé à un microprocesseur 4 qui peut être du type PIC16C54 qui est le plus bas de la gamme de tels microprocesseurs.

Le microprocesseur 4 comporte quatre bornes auxquelles sont reliés quatre contacts 5 au moyen desquels l'utilisateur peut sélectionner l'une quelconque des différentes fréquences produites par le microprocesseur 4.

L'appareil est capable de produire des ondes ou oscillations dont les fréquences peuvent aller jusqu'à la plage des mégahertz. Cependant dans le cas présentement considéré, peuvent se trouver comprises dans la gamme des ondes pouvant atteindre les trente hertz (30 Hz), ces ondes pouvant avoir des valeurs particulières de leurs fréquences de 161Hz, 2564Hz, 7832Hz, 8216Hz et 22453Hz, pour les fréquences sélectionnables au moyen des différents contacts 5 du microprocesseur 4, et ce de telle façon que lorsque les quatre contacts 5 sont ouverts, la valeur de 161Hz soit sélectionnée et qu'en fonction de la fermeture des quatre contacts 5, les quatre autres valeurs restantes se trouvent être respectivement sélectionnées.

Les signaux qui sont ainsi produits, sont diffusés par l'intermédiaire d'une antenne émettrice formée par une bobine 6 qui est reliée à la sortie du microprocesseur 4 par la résistance 7 limitant le courant.

Le champ d'action de l'émission électromagnétique est déterminé par la taille de l'antenne 6 qui, en accord avec la dimension réduite de l'appareil, est d'un type qui permet de produire un champ électrique capable de couvrir un volume de l'espace compris entre un et deux mètres cubes, ce qui donne la possibilité d'utiliser l'appareil aussi bien en le laissant sur l'utilisateur qu'en le plaçant à proximité des lieux d'activité ou de repos.

Grâce aux contacts 5 on peut sélectionner les différentes fréquences suivant l'objectif recherché dans chaque cas. A ces contacts 5, qui sont formés par exemple par des micro-interrupteurs, est relié un interrupteur traditionnel, non représenté, qui assume la fonction de connexion et de déconnexion de l'alimentation électrique, afin de mettre en service ou hors service l'appareil, de telle façon que, lors de la mise en service, l'une des fréquences, par exemple la fréquence la plus basse de l'ensemble de fréquences par suite du fait que tous les contacts 5 sont ouverts, soit sélectionnée tandis qu'en fermant les contacts 5, on peut sélectionner les autres fréquences, l'interrupteur de mise en service devant être toujours fermé, dans chaque cas, pour que l'appareil fonctionne.

Il est prévu que, lorsque l'appareil est mis en service et chaque fois que l'on change la fréquence émise, l'opération soit signalée, pendant un certain temps, par suite de l'allumage d'un afficheur traditionnel à diode électroluminescente, non représenté, ce qui permet de vérifier l'effectivité du changement opéré ou de la connexion de mise en service suivant le cas. En outre, si, au moment de la mise en service ou d'un changement de fréquence, cet afficheur ne s'allume pas, ceci indique que la pile ou la batterie d'alimentation est épuisée.

## Revendications

1. Générateur d'ondes électromagnétiques **caractérisé en ce qu'**il comprend un microprocesseur (4) avec un circuit oscillant formé par un cristal de quartz (1) et deux condensateurs (2,3), et, à la sortie de ce microprocesseur (4), une antenne émettrice formée par une bobine (6) laquelle est directement reliée à la sortie du microprocesseur (4) par l'intermédiaire d'une résistance limitatrice de courant (7).

2. Générateur d'ondes électromagnétiques suivant la revendication 1 **caractérisé en ce que** le microprocesseur (4) comporte une série de contacts-interrupteurs (5) au moyen desquels peut être sélectionnée l'activation du microprocesseur (4), en vue de la production d'ondes de différentes fréquences à partir de la fréquence d'horloge que produit le circuit oscillant.

3. Générateur d'ondes électromagnétiques suivant la revendication 2 **caractérisé en ce que** le microprocesseur (4) est pourvu d'un afficheur lumineux à allumage temporaire qui s'allume au moment de la mise en service du générateur et de l'actionnement de l'un quelconque des contacts-interrupteurs (5).

## Patentansprüche

1. Generator für elektromagnetische Wellen, **gekennzeichnet durch** einen Mikroprozessor (4) mit einem **durch** einen Quarzkristall (1) und zwei Kondensatoren (2, 3) gebildeten Schwingkreis und eine am Ausgang des Mikroprozessors (4) vorgesehene Sendeantenne, die **durch** eine Spule (6) gebildet ist, welche über einen Strombegrenzungswiderstand (7) direkt mit dem Ausgang des Mikroprozessors (4) verbunden ist.

2. Generator für elektromagnetische Wellen nach Anspruch 1, **dadurch gekennzeichnet, dass** der Mikroprozessor (4) mit einer Reihe von Schaltkontakten (5) versehen ist, mittels derer er zur Erzeugung von Wellen unterschiedlicher Frequenzen, basierend auf der vom Schwingkreis erzeugten Taktfrequenz, aktivierbar ist.

3. Generator für elektromagnetische Wellen nach Anspruch 2, **dadurch gekennzeichnet, dass** der Mikroprozessor (4) mit einer vorübergehend leuchtenden Leuchtanzeige versehen ist, die im Moment der Inbetriebnahme des Generators und bei Betätigung eines der Schaltkontakte (5) aufleuchtet.

## Claims

1. Generator of electro-magnetic waves, **characterized in that** it comprises a microprocessor (4) with an oscillatirng circuit formed by a quartz crystal (1) and two capacitors (2, 3) and, at the output of this microprocessor (4), an emitter antenna formed by a coil (6) which is directly connected to the output of the microprocessor (4) via a current limiting resistor (7).

2. Generator of electro-magnetic waves according to Claim 1, **characterized in that** the microprocessor (4) comprises a series of contact switches (5) by means of which the activation of the microprocessor (4) may be selected, with a view to the production of waves of different frequencies from the clock frequency that the oscillating circuit produces.

3. Generator of electro-magnetic waves according to Claim 2, **characterized in that** the microprocessor (4) is provided with a light displaying means with temporary illumination which lights up at the moment when the generator is switched on and any one of the contact switches (5) is actuated.
